(19) Europäisches Patentamt
European Patent Office
Office européen des brevets

(11) EP 1 069 898 B1

(12) EUROPEAN PATENT SPECIFICATION

(45) Date of publication and mention of the grant of the patent:
**12.05.2004 Bulletin 2004/20**

(21) Application number: **99908574.9**

(22) Date of filing: **01.03.1999**

(51) Int Cl.⁷: **A61K 31/45**, A61P 31/02

(86) International application number:
**PCT/US1999/004412**

(87) International publication number:
**WO 1999/047140 (23.09.1999 Gazette 1999/38)**

# (54) TOPICAL ANTISEPTIC COMPOSITIONS AND METHODS

TOPISCHE ANTISEPTISCHE ZUSAMMENSTELLUNGEN UND METHODEN

COMPOSITIONS ET METHODES TOPIQUES ANTISEPTIQUES

(84) Designated Contracting States:
**AT BE CH CY DE DK ES FI FR GB GR IE IT LI LU MC NL PT SE**

(30) Priority: **17.03.1998 US 78307 P**

(43) Date of publication of application:
**24.01.2001 Bulletin 2001/04**

(73) Proprietor: **MARGOLIN, Solomon B.**
**Dallas, Texas 75225 (US)**

(72) Inventor: **MARGOLIN, Solomon B.**
**Dallas, Texas 75225 (US)**

(74) Representative: **Harrison, Ivor Stanley et al**
**Withers & Rogers,**
**Goldings House, 2 Hays Lane**
**London SE1 2HW (GB)**

(56) References cited:
**WO-A-96/27374**
**WO-A-97/10712**
**WO-A-97/41830**
**US-A- 3 839 346**
**US-A- 3 974 281**
**US-A- 5 179 098**
**US-A- 5 314 892**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 069 898 B1

## Description

### Technical Field

The present invention relates to antiseptic compositions and, more particularly, to an ointment, cream, or foam containing pirfenidone and/or related compounds, for disinfecting the skin.

### Background Art

A large number of various disinfecting preparations are known. When hygienic purposes are concerned, disinfection becomes rather difficult, because it is necessary to reconcile an efficient antiseptic effect with demonstrable harmlessness with respect to the skin. Many such known disinfecting preparations cause adverse reactions when applied to the skin, such as skin irritation, even though the preparations may have satisfactory antiseptic properties.

Accordingly, it is a principal object of the present invention to provide new disinfectant compositions and methods of application which are harmless to the skin, while exerting a marked killing action on microorganisms which develop on or in the outer layers of the dermis.

It is a further object of the invention to provide such a disinfectant composition as an ointment, cream, or foam formed in an aqueous dispersion of one or more substances, the water of the dispersion being dissolved or emulsified therein and carrying suitable fatty solvents and an active ingredient.

It is an additional object of the invention to provide such a disinfectant composition that is self-sanitizing, that is, the composition causes the complete destruction of bacteria and fungi after the composition has been deliberately inoculated with contaminating infectious microbes.

It is another object of the invention to provide such a disinfectant composition that can be utilized as an antimicrobial disinfectant for various inanimate objects.

It is yet a further object of the invention to provide such a disinfectant that is long lasting because of the persistence of the active agent on the surface of the skin.

It is yet a an additional object of the invention to provide such a disinfectant that penetrates into the several outer layer of the dermis.

It is yet another object of the invention to provide such a disinfectant that is harmless with respect to the skin.

Other objects of the present invention, as well as particular features, elements, and advantages thereof, will be elucidated in, or be apparent from, the following description and the accompanying drawing figures.

W096/27374 describes the use of N-substituted 2(1H) pyridones and 3(1H) pyridones to block or inhibit the activity of certain cytokine growth factors to treat a range of conditions involving proliferation of mesenchymal cells.

According to the present invention, there is provided the use of one or more 2(1H) pyridone compounds in the preparation of a topical pharmaceutical composition for use as an antiseptic.

### Best Mode for Carrying Out the Invention

The present invention relates to the use of medical compositions for the novel antiseptic topical treatment of microbial (bacteria, fungi, viruses) on the surface of, or within, the layers of the dermis of skin, ears, fingernails, toenails, or hoofs of mammalian species, which composition comprises pirfenidone (5-methyl-1-phenyl-2-(1H) pyridone) and/or related compounds in an appropriate formulation of a pharmaceutical topical ointment, cream, lotion, or solution.

Furthermore, the present invention is directed to the use of a non-irritating, emollient, germicidal pharmaceutical composition. This composition affords a method of using the formulations as antiseptics for untoward skin conditions such as a dermal wound, bruise, or microbial infections, as well as for other damaged external body surface tissues, by safely penetrating outer layers of skin and related tissues or structures. In addition, the antimicrobial data indicate the compositions to be self-sanitizing, since the composition can cause marked elimination or complete destruction of bacteria and fungi after the composition has been deliberately inoculated with infectious microbes. The composition also can be utilized as an antimicrobial disinfectant for various inanimate objects. As formulated, the composition is stable for many months at temperatures of 25 degrees Centigrade or less.

The invention provides an ointment, cream, or foam formed in an aqueous dispersion of one or more active substances, the water of the dispersion being dissolved or emulsified therein and carrying suitable fatty solvents and the active ingredient(s), pirfenidone and/or related compounds. The preferred concentration of the active ingredient(s) is from about 5 to about 10 weight percent.

The composition of the present invention provides an effective microbial sanitizer, disinfectant, and barrier in one composition. The antimicrobial effects are very marked as evidenced by the several tests set forth below.

By adding suitable ingredients to the active compound(s), such as glycerine, natural colorants, surfactants, emulsifiers, and oils, it is possible to produce antiseptic products which are presented as antiseptic lotions, antiseptic

solutions, lotions, antiseptic ointments, and antiseptic foams. In the case of antiseptic ointments, creams, lotions, and solutions, the cited bactericidal, fungicidal, and virucidal effects are recognizable within minutes. The antiseptic products and their method of manufacture are illustrated below.

The ingredients of the compositions include USP products: white petrolatum, propylene glycol 400, and stearyl alcohol, for example, all appropriately dissolved and emulsified into purified distilled water. In order to illustrate the general dermatological composition of the present invention, a base composition of the following can be prepared:

| MODIFIED USP HYDROPHILIC OINTMENT (5.0% PIRFENIDONE) | |
|---|---|
| Ingredient | Percent by Weiqht |
| Pirfenidone (USAN) Powder | 5.0 |
| Propylene Glycol 400 USP | 23.5 |
| Sterile Distilled Water | 30.5 |
| Stearyl Alcohol USP | 20.5 |
| White Petrolatum USP | 20.5 |
| | TOTAL: 100.0 |

| MODIFIED USP HYDROPHILIC OINTMENT (10.0% PIRFENIDONE) | |
|---|---|
| Ingredient | Percent by Weight |
| Pirfenidone (USAN) Powder | 10.0 |
| Propylene Glycol 400 USP | 14.3 |
| Sterile Distilled Water | 41.7 |
| Stearyl Alcohol USP | 17.0 |
| White Petrolatum USP | 17.0 |
| | TOTAL: 100.0 |

It is important to mix and melt the aqueous components (pirfenidone, propylene glycol 400, and water), heating to about 70 degrees Centigrade independently from the lipophilic phases (stearyl alcohol and white petrolatum) which also must be heated to about 70 degrees Centigrade to facilitate mixing and melting. When each phase has been adequately mixed and melted, they are combined and cooled with rapid stirring, until the mixture congeals into a fluffy, white ointment. The temperature of the ointment when it congeals will be about 40-45 degrees Centigrade. (Failure to adequately mix by vigorous stirring during the chilling step will result in the separation of the two solvent phases, and the emulsifying properties of the formulation will have been lost.)

A critical feature of the compositions of this invention is the chemical stability of the active ingredient(s), pirfenidone and/or related compounds. Crystalline pirfenidone is stable at room temperature (i.e., 25 degrees Centigrade) for more than five years. The formulations described above are stable for two years or longer at 25 degrees Centigrade (room temperature), based upon chemical assays and upon physical characteristics (color, plasticity, active ingredient dispersion and suspension).

The formulations described above have demonstrated their efficacy against the following microorganisms:

Escherichia coli
Staphylococcus aureus
Bacillus subtilis
Pseudomonas aeruginosa
Proteus vulgaris
Trichophyton mentagorphytes
Candida albicans
Aspergillis niger
Influenza virus
Coxsakie virus
Herpes virus
Papilloma virus

TABLE I

| PRELIMINARY ANTIMICROBIAL TEST OF PIRFENIDONE | | | | | | |
|---|---|---|---|---|---|---|
| | Pirfenidone Concentrations, wt.% | | | | | |
| 2.0 | | 0.0* | 5.0 | 0.0* | 10.0 | 0.0* |
| | GROWTH SCORES (0 TO 10)*** | | | | | |
| BACTERIA: | | | | | | |
| Proteus vulgaris | -** | - | 0 | 10 | 0 | 10 |
| Escherichia coli | - | - | 0 | 10 | 0 | 10 |
| Pseudomonas aeruginosa | 1 | 10 | 0 | 10 | 0 | 10 |
| Bacillus subtilis | - | - | 0 | 10 | 0 | 10 |
| Staphylococcus aureus - | | - | 0 | 10 | 0 | 10 |
| FUNGI: | | | | | | |
| Candida albicans | - | - | 2 | 10 | 0 | 10 |
| Aspergillus niger | - | - | 0 | 10 | 0 | 10 |
| Trychophton mentagrophytes | - | - | 2 | 10 | 0 | 10 |

* Control.

** Not tested.

*** 1=very slight
2=slight
3=slight to moderate
4=moderate
10=total plate growth (maximal growth)

METHODS FOR MEASURING ANTIMICROBIAL ACTIVITY IN TOPICAL PREPARATIONS FOR TABLE I:

TESTING DISINFECTING ACTIVITY

A nutritive broth is prepared by dissolving a commercial nutritive substance in 1000 ml. of sterile distilled water. The solution is heated to 100 degrees Centigrade and poured into sterile Petri dishes under sterile conditions. After cooling and solidifying, the gels are then kept at 37 degrees Centigrade for the specified number of hours or days.

Using the modified USP hydrophilic ointment with and without pirfenidone, the procedure outlined below was followed to determine bacterial and fungal counts. This procedure is based on that described in the booklet "microbiological Examination of Topical Drugs and Cosmetics," published by the Division of Microbiology, United States Food and Drug Administration, January 7, 1969.

Bacteria Plate Count:

Ten (10) grams of sample is aseptically measured into 90 ml. diluent (Butterfield's phosphate diluent with azolectin and Tween 80) to make a 10 (1 pwr) dilution. Decimal dilutions from 10 (1 pwr) to 10 (4 pwr) are made using 90 ml. dilution blanks of Butterfield's phosphate diluent. Duplicate plates from each of the above dilutions are made following directions of AOAC, 11th ed., 1970, pp 842-843, 41.015, except for the use of Trypticase Soy Agar (42-45 degrees Centigrade) in place of plate count agar.

One ml. of each dilution is placed into a Petri dish and Trypticase Soy Agar is added within 15 minutes from the time of original dilution. Plates were incubated for 48 hours at 35 degrees Centigrade, and duplicate plates for each dilution with colony counts in the range of 30 to 300 per plate are counted and averaged. Counts are reported as aerobic plate count per gram of sample.

Fungi Plate Count:

Decimal dilutions as described above for the aerobic bacteria plate count are prepared. Aliquots of 1.0 ml. of each dilution are delivered to each of quadruplicate (4) plates. Plates are poured with 20-25 ml. of "Sabouraud's Dextrose Agar. Two plates are incubated at 37 degrees Centigrade, and other two plates are incubated at room temperature

(26 degrees Centigrade) for seven days. Counts of duplicate plates are averaged and reported, in each case, as counts per gram of sample.

TABLE II

| ADDITIONAL PIRFENIDONE ANTI-MICROBIAL PILOT TESTS | | |
|---|---|---|
| Test # 1:. | | |
| A 2.0% solution of pirfenidone was prepared in nutrient broth, and then was inoculated with Pseudomonas aeruginosa. After 48 hours of incubation at 37 degrees Centigrade, the nutrient broth failed to evidence any growth. Then a standard loopful from this pirfenidone treated broth was streaked on Tryptic Soy Agar (Difco) and incubated for 5 days at 37 degrees Centigrade; no growth of Pseudomonas aeruginosa was seen. | | |
| Test # 2: | | |
| A 5.0% solution of pirfenidone in Tryptic Soy Agar (Difco) was formulated. Pirfenidone was dissolved in hot agar. When permitted to cool to room temperature (26 degrees Centigrade), pirfenidone became a suspension in a uniform slightly opaque manner throughout the agar. At 10.0% of pirfenidone in Tryptic Soy Agar, the drug formed a uniformly opaque suspension in the agar. Growth was completely inhibited at both concentrations of the following organisms: | | |
| Antiseptic Effect Against Bacteria: | | |
| Agar inoculated with the following bacteria, and incubated at 37 degrees Centigrade: | | |
| Escherichia coli | ATCC | #11229 |
| Proteus vulgaris | ATCC | # 6538 |
| Bacillus subtilis | ATCC | #19659 |
| Staphylococcus aureus | ATCC | #13315 |
| Pseudomonas aeruginosa | ATCC | #15442 |
| Antiseptic Effect Against Fungi: | | |
| Agar inoculated with the following fungi and incubated at 26 degrees Centigrade: | | |
| Trichophyton mentagrophytes | ATCC | # 9129 |
| Candida albicans | ATCC | #10259 |
| Asperqillis niger | ATCC | # 9642 |

TABLE III

**CHALLENGE TESTS**

Challenge tests were conducted of pirfenidone against microbial inoculations into: (a) nutrient broth, and (b) 5.0% or 10.0% pirfenidone ointments (modified USP hydrophilic ointment). A Pseudomonas aeruginosa inoculation into broth served as a positive control.

The bacterial mixture for inoculation consisted of:

- Escherichia coli
- Proteus vulgaris
- Bacillus subtilis
- Staphylococcus aureus
- Pseudomonas aeruginosa

The fungal mixture for inoculating consisted of:

- Trichophyton mentagrophytes
- Candida albicans
- Aspergillis niger

After seven days, the respective cultures were plated out to determine the number of microbes present. The results as compared with the baseline number of microbes present when the cultures were inoculated follows:

| | Pseudomonas (Positive Control) | Mixed Bacteria | Mixed Fungi |
|---|---|---|---|
| | Microbes per gm. of Sample (After 7 days) | | |
| Baseline (day 1) (No Pirfenidone) | 52 million (100.0%) | 23 million (100.0%) | 240,000 (100.0%) |
| 2.0% Broth Solution (Pirfenidone) | <100 (0.0%) | 530,000 (2.3%) | 2,000 (0.08%) |
| 5.0% Ointment (Pirfenidone) | 510,000 0.01%) | 57,000 (0.02%) | 350 (0.002%) |
| 10.0% Ointment ( Pirfenidone) | 2,600 (0.0001%) | 70,000 (0.03%) | <10 (0.0%) |

TABLE IV

CHALLENGE EXPERIMENTS

Challenge experiments were conducted over four weeks for pirfenidone ointment against mixed microbial inoculations into: (a) nutrient broth, and (b) 5.0% or 10.0% pirfenidone ointments. A Pseudomonas aeruginosa inoculation into broth served as a positive control.

The bacterial mixture for inoculation consisted of:

| | | |
|---|---|---|
| Escherichia coli | ATCC | #11229 |
| Proteus vulgaris | ATCC | # 6538 |
| Bacillus subtilis | ATCC | #19659 |
| Staphylococcus aureus | ATCC | #13315 |
| Pseudomonas aeruginosa | ATCC | #15442 |

The fungal mixture for inoculation consisted of:

| | | |
|---|---|---|
| Trichophyton mentagrophytes | ATCC | # 9129 |
| Candida albicans | ATCC | #10259 |
| Aspergillis niger | ATCC | # 9642 |

At weekly intervals for four weeks, the respective cultures were plated out into Petri dishes to determine the number of microbes present. The results as compared with the baseline number of microbes present when the cultures were first inoculated follow:

| | Pseudomonas (Positive Control) | Mixed Bacteria | Mixed Fungi |
|---|---|---|---|
| Baseline (day 1) (No Pirfenidone) | 52 million (100.0%) | 23 million (100.0%) | 240,000 (100.0%) |
| **2.0% Broth Solution (Control) (With Pirfenidone)** | | | |
| After 1 week: | <100 (0.0%) | 590,000 (2.3%) | <100 (0.0%) |
| After 2 weeks: | <10 (0.0%) | 690,000 (3.0%) | <10 (0.0%) |
| After 3 weeks: | <10 (0.0%) | 1,100,000 (4.4%) | <10 (0.0%) |
| After 4 weeks: | <10 (0.0%) | 510,000 (2.2%) | <10 (0.0%) |
| **5.0% Ointment (Pirfenidone)** | | | |
| After 1 week: | 510,000 (0.98%) | 57,000 (0.25%) | <100 (0.0%) |
| After 2 weeks: | 1,480,000 (2.8%) | 68,000 (0.30%) | <10 (0.0%) |
| After 3 weeks: | 790,000 (1.5%) | 65,000 (0.28%) | <100 (0.0%) |
| After 4 weeks: | 180,000 (0.34%) | 44,000 (0.19%) | <10 (0.0%) |
| **10.0% Ointment (Pirfenidone)** | | | |
| After 1 week: | 260,000 (0.50%) | 70,000 (0.30%) | <10 (0.0%) |
| After 2 weeks: | 2,060,000 (3.9%) | 166,000 (0.71%) | <10 (0.0%) |
| After 3 weeks: | 1,240,000 (2.2%) | 280,000 (1.20%) | <10 (0.0%) |
| After 4 weeks: | 1,020,000 | 131,000 | <10 |

| | | | |
|---|---|---|---|
| | (2.0%) | (0.57%) | (0.0%) |

(End of Table IV)

The data (Tables I, II, III, and IV) demonstrate that compositions (solutions or ointments) containing pirfenidone at concentrations of 2.0% to 10.0% are distinctly disinfective against aerobic pathogenic bacteria and fungi in a manner typical of antiseptics, and their efficacy increases within a range of increasing concentrations. Disinfectant effects are greatly reduced at concentrations lower than 1.5%.

SAFETY

Primary Skin Irritation Tests:

According to several primary skin (abraded and non-abraded) irritant test in albino rabbits, the primary irritation index is well below 0.5, and therefore the tests samples of the respective compositions cannot be classified as positive skin irritants.

Acute Topical Irritation/Local Toxicity Tests:

(1) Primary Rabbit Acute Eye Irritation Test for 2.0% Pirfenidone Solution

Pirfenidone, as a 2.0% sterile aqueous solution was applied to the eye corneas of six albino rabbits, and failed to cause any irritation as evidenced by the absence of hyperemia, edema, eye discomfort, or chemotaxis (Draize method). The eyes were carefully examined at 1.0 minutes, 30 minutes, and 3 hours after applying the solution, and then repeatedly examined for 10 days after the application of the pirfenidone solution (0.1 ml. per eye).

(2) Primary Rabbit Acute Eye Irritation Test for 10.0% Pirfenidone Modified USP Hydrophilic Ointment

Modified USP hydrophilic ointment containing 10.0% pirfenidone was applied to the right eye corneas of 6 albino rabbits and did not cause any irritation as evidenced by the absence of hyperemia, edema, eye discomfort, or chemotaxis (Draize method). The eyes were carefully examined at 1, 3, 8, 24, and 48 hours and carefully re-examined daily for 10 days after the application of the ointment (100 milligrams per eye).

(3) Subacute (21 days) Dermal Local and Systemic Toxicity in Albino Rabbits

Graded amounts of modified USP hydrophilic ointment containing 10.0% pirfenidone repeatedly was topically applied to the dorsal aspect of the clipped abraded or non-abraded skin of the back. The graded amounts of ointment were 200 mg/kg/day, 2000 mg/kg/day, and 5000 mg/kg/day. The controls received 5000 mg/kg/day of the placebo vehicle ointment. The rabbits were observed carefully each day for signs of any irritation to the skin (erythema, edema, necrosis, etc.) and were scored according to the method of Draize. They also were observed for any alterations in general appearance and behavior.

No evidence of irritation of the skin (abraded or non-abraded) was seen in any of the groups. No effect was seen at any dose level upon general appearance, behavior, body weight gain, or upon any of the detailed hematological and blood chemistry values, or upon urinalyses. In addition, gross and histopathological examination of several vital organs and tissues failed to show any drug-related changes. In this subacute rabbit experiment, the data indicates that 5000 mg/kg/day of a 10.0% pirfenidone ointment for 21 days is devoid of any demonstrable local or systemic toxicity.

ACUTE ORAL TOXICITY OF 10.0% PIRFENIDONE HYDROPHILIC OINTMENT IN RODENTS

The acute toxicity of the composition cited, determined in female and male rats and/or mice, exceeds 5000 mg/kg/day when administered orally, or topically.

In fasted albino mice, the oral LD50 was calculated to be 11,000 +/- 1,100 mg/kg of body weight. This was determined according to the mortality found over 14 days following administration of the six graded doses to groups of 7 mice per dose level. In fasted albino rats, the oral LD50 was greater than 10,000 mg/kg body weight, since no deaths and no signs of toxicity occurred.

The following are illustrative examples of the various end use compositions of the invention.

EXAMPLE 1

A modified USP hydrophilic ointment composition was prepared containing, however, 10.0% of pirfenidone. The composition was applied topically to patients who recently experienced lacerations which had become infected. No systemic antimicrobial agents were used. Remission of the infection occurred within 24 hours and complete healing occurred within 5 to 10 days.

EXAMPLE 2

Modified USP hydrophilic ointment composition was prepare containing 10.0% of pirfenidone. The composition was applied topically daily to the toenails of patients with longstanding (several years) fungus infections of the toenails. These infections had been treated repeatedly with many types of antifungal agents without success. The pirfenidone ointment completely cleared these fungus infections in 3 to 12 weeks, and the lesions did not recur on 2-year follow-ups. Pirfenidone is unusual in its ability to penetrate into the collagenous matrix of the toenail, and then into the dermal layers underneath the toenail.

The cited hydrophilic ointment also is very effective in successfully treating, as well as preventing, the fungus infections characteristic of "athlete's foot".

EXAMPLE 3

The composition was prepared and applied topically to patients having a bacterial infection and inflamed local rash at a rate of three times daily. Relief of discomfort occurred within 1 to 3 hours, and complete healing along with clearing of the rash, was seen after 5 to 7 days.

EXAMPLE 4

The above cited hydrophilic composition was applied to patients having debraded skin due to a scalding burn. Improvement included reduced irritation within one hour and a marked remission was seen in 24 hours after commencement of treatment and subsequently no infections occurred. The composition was applied 3 times daily until full remission was achieved. Complete remission was present after 5 to 7 days.

EXAMPLE 5

In vivo with patients. Intact or ruptured blisters and sharp soreness of "cold sores" (herpes virus #1) on lips and adjacent oral regions of skin were terminated readily after topical daily applications of 10.0% pirfenidone hydrophilic ointment, and the lesions were gone in 5 to 10 days.

In vivo with patients. Various dermal facial warts (papilloma viruses) were eliminated by repeated daily applications of 10.0% pirfenidone hydrophilic ointment, and the warts were gone in 3 to 6 weeks after initiating treatment with the ointment depending on the size of the wart.

EXAMPLE 6

As a barrier ointment or cream, pirfenidone hydrophilic ointment prevents the re-infection of previously treated microbial lesions, and has been repeatedly been observed in patients with various dermal cuts, traumatic injuries, and also in bed-ridden patients with "bed sores".

EXAMPLE 7

An example of a modified USP hydrophilic ointment (10.0% pirfenidone) is as follows:

| | |
|---|---|
| Pirfenidone (USAN) Powder | 100 gms |
| Propylene Glycol 400 USP | 143 ml (143 gms) |
| Sterile Distilled Water | 417 ml (417 gms) |
| Stearyl Alcohol USP | 170 gms |
| White Petrolatum | 170 gms |
| TOTAL: | 1000 gms |

EXAMPLE 8

An example of a vanishing cream formula (5.0% pirfenidone) is as follows:

| | |
|---|---|
| Pirfenidone (USAN) Powder | 50 gms |
| Stearic Acid USP | 30 gms |
| Emplilan SE 40* | 30 gms |
| Isopropyl Myristate | 30 gms |
| Mineral Oil | 115 gms |
| Stearyl Alcohol USP | 5 gms |
| Propylene Glycol 400 USP | 50 gms |
| Sterile Distilled Water | 690 ml (690 gms) |
| TOTAL: | 1000 gms |

* Trademark

Methods of preparation of pirfenidone and related compounds are described in US Patent No. 3,839,346, issued October 1, 1974, to Gadekar, and titled N-SUBSTITUTED PYRIDONES AND GENERAL METHOD FOR PREPARING PYRIDONES.

In addition to pirfenidone, 2-(1H) pyridone compounds having the following general structural formula have been shown to, or are expected to, have the same antiseptic properties, when applied in the concentrations and vehicles as described above with respect to pirfenidone:

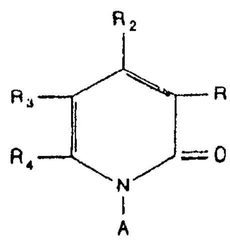

where: R1 = alkyl group (CH3, C2H5, etc.); A is phenyl, thienyl, etc., or other aryl group. The alternate is for R3 to be the site of substitution of the alkyl group with R1 remaining as a hydrogen; R2 and R4 are, in every circumstance, hydrogens.

Examples of the additional 2-(1H) pyridones include:

5-Methyl-1-(3-nitrophenyl-2)-(1H) pyridone
5-Methyl-1-(4'-methoxyphenyl)-2-(1H) pyridone
5-Methyl-1-p-tolyl-2-(1H) pyridone
5-Methyl-1-(3'-trifluoromethylphenyl)-2-(1H) pyridone
1-(4'Chlorophenyl)-5-Methyl-2-(1H) pyridone
5-Methyl-1-(2'-naphthyl)-2-(1H) pyridone
5-Methyl-1-(1'naphthyl)-2-(1H) pyridone
3-Methyl-1-phenyl-2-(1H) pyridone
3-Ethyl-1-phenyl-2-(1H) pyridone
6-Methyl-1-phenyl-2-(1H) pyridone
3,6-Dimethyl-1-phenyl-2-(1H) pyridone
5-Methyl-1-(2'-Thienyl)-2-(1H) pyridone
1-(2'-Furyl)-5-Methyl-2-(1H) pyridone
5-Methyl-1-(5'-quinolyl)-2-(1H) pyridone
5-Methyl-1-(4'-pyridyl)-2-(1H) pyridone
5-Methyl-1-(3'-pyridyl)-2-(1H) pyridone
5-Methyl-1-(2'-pyridyl)-2-(1H) pyridone
5-Methyl-1-(2'-quinolyl)-2-(1H) pyridone
5-Methyl-1-(4'-quinolyl)-2-(1H) pyridone

5-Methyl-1-(2'-thiazolyl)-2-(1H) pyridone
1-(2'-Imidazolyl)-5-Methyl-2-(1H) pyridone
5-Ethyl-1-phenyl-2-(1H) pyridone
1-Phenyl-2-(1H) pyridone
1-(4'-Nitrophenyl)-2-(1H) pyridone
1,3-Diphenyl-2-(1H) pyridone
1-Phenyl-3-(4'-chlorophenyl)-2-(1H) pyridone
1,3-Diphenyl-5-methyl-2-(1H) pyridone
3-(4'-Chlorophenyl)-5-Methyl-1-phenyl-2-(1H) pyridone, and
5-Methyl-3-phenyl-1-(2'-thienyl)-2-(1H) pyridone.

## Claims

**1.** Use of one or more 2-(1H) pyridone compound(s) in the preparation of a topical pharmaceutical composition for use as an antiseptic.

**2.** A use according to claim 1, in which the one or more 2-(1H) pyridone compound(s) is (are) present in an ointment, cream, or foam.

**3.** A use according to claim 2, in which said one or more 2-(1H) pyridone compound(s) is (are) present in an aqueous dispersion of one or more substances.

**4.** A use according to any preceding claim, in which said one or more 2-(1H) pyridone compounds are present in a concentration of from about two weight percent to about 10 weight percent of the composition.

**5.** A use according to any preceding claim, in which said one or more 2-(1H) pyridone compounds have the following general structural formula:

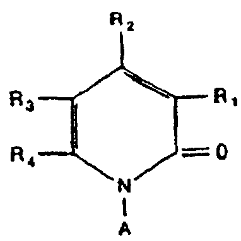

wherein $R_1$ is an alkyl group; A is phenyl, thienyl or other aryl group; alternatively, $R_3$ is the site of substitution of said alkyl group with $R_1$ remaining as a hydrogen; and $R_2$ and $R_4$ are, in every circumstance, hydrogens.

**6.** A use according to claim 5, in which said one or more 2-(1H) pyridone compounds are selected from the group consisting of:

5-Methyl-1-phenyl-2-(1H) pyridone
5-Methyl-1-(3-nitrophenyl-2)-(1H) pyridone
5-Methyl-1-(4'-methoxyphenyl)-2-(1H) pyridone
5-Methyl-1-p-tolyl-2-(1H) pyridone
5-Methyl-1-(3'-trifluoromethylphenyl)-2-(1H) pyridone
1-(4'Chlorophenyl)-5-Methyl-2-(1H) pyridone
5-Methyl-1-(2'-naphthyl)-2-(1H) pyridone
5-Methyl-1-(1'naphthyl)-2-(1H) pyridone
3-Methyl-1-phenyl-2-(1H) pyridone
3-Ethyl-1-phenyl-2-(1H) pyridone
6-Methyl-1-phenyl-2-(1H) pyridone
3,6-Dimethyl-1-phenyl-2-(1H) pyridone
5-Methyl-1-(2'-Thienyl)-2-(1H) pyridone

1-(2'-Furyl)-5-Methyl-2-(1H) pyridone
5-Methyl-1-(5'-quinolyl)-2-(1H) pyridone
5-Methyl-1-(4'-pyridyl)-2-(1H) pyridone
5-Methyl-1-(3'-pyridyl)-2-(1H) pyridone
5-Methyl-1-(2'-pyridyl)-2-(1H) pyridone
5-Methyl-1-(2'-quinolyl)-2-(1H) pyridone
5-Methyl-1-(4'-quinolyl)-2-(1H) pyridone
5-Methyl-1-(2'-thiazolyl)-2-(1H) pyridone
1-(2'-Imidazolyl)-5-Methyl-2-(1H) pyridone
5-Ethyl-1-phenyl-2-(1H) pyridone
1-Phenyl-2-(1H) pyridone
1-(4'-Nitrophenyl)-2-(1H) pyridone
1,3-Diphenyl-2-(1H) pyridone
1-Phenyl-3-(4'-chlorophenyl)-2-(1H) pyridone
1,3-Diphenyl-5-methyl-2-(1H) pyridone
3-(4'-Chlorophenyl)-5-Methyl-1-phenyl-2-(1H) pyridone, and
5-Methyl-3-phenyl-1-(2'-thienyl)-2-(1H) pyridone.

**7.** A use according to any preceding claim, in which said pharmaceutical composition has the following composition, in weight percent:

| | |
|---|---|
| Pirfenidone (USAN) Powder | 5.0 |
| Propylene Glycol 400 USP | 23.5 |
| Sterile Distilled Water | 30.5 |
| Stearyl Alcohol USP | 20.5 |
| White Petrolatum USP | 20.5 |
| | TOTAL: 100.0 |

**8.** A use according to any of claims 1 to 6, in which said pharmaceutical composition has the following composition, in weight percent:

| | |
|---|---|
| Pirfenidone (USAN) Powder | 10.0 |
| Propylene Glycol 400 USP | 14.3 |
| Sterile Distilled Water | 41.7 |
| Stearyl Alcohol USP | 17.0 |
| White Petrolatum USP | 17.0 |
| | TOTAL: 100.0 |

**9.** A use according to any of claims 1 to 6, in which said pharmaceutical substance has the following composition, in weight percent:

| | |
|---|---|
| Pirfenidone (USAN) Powder | 50 gms |
| Stearic Acid USP | 30 gms |
| Emplilan SE 40* | 30 gms |
| Isopropyl Myristate | 30 gms |
| Mineral Oil | 115 gms |
| Stearyl Alcohol USP | 5 gms |
| Propylene Glycol 400 USP | 50 gms |
| Sterile Distilled Water | 690 ml (690 gms) |
| | TOTAL: 1000 gms |

**10.** A use according to any preceding claim, in which the composition is for treating infections caused by bacteria, fungi, and/or viruses on the surface of, or within, the layers of the dermis of skin, ears, fingernails, toenails, or hoofs'of mammalian species.

**Patentansprüche**

1. Verwendung einer oder mehrerer 2-(1H)-Pyridon-Verbindung(en) bei der Herstellung einer topischen pharmazeutischen Zusammensetzung für die Verwendung als ein Antiseptikum.

2. Verwendung nach Anspruch 1, wobei die eine oder die mehreren 2-(1H)-Pyridon-Verbindung(en) in einer Salbe, einer Creme oder einem Schaum vorhanden ist (sind).

3. Verwendung nach Anspruch 2, wobei die eine oder die mehreren 2-(1H)-Pyridon-Verbindung(en) in einer wässrigen Dispersion aus einer oder mehreren Substanzen vorhanden ist (sind).

4. Verwendung nach einem der vorherigen Ansprüche, wobei die eine oder die mehreren 2-(1H)-Pyridon-Verbindungen in einer Konzentration von etwa 2 Gewichtsprozent bis etwa 10 Gewichtsprozent der Zusammensetzung vorhanden sind.

5. Verwendung nach einem der vorherigen Ansprüche, wobei die eine oder die mehreren 2-(1H)-Pyridon-Verbindungen die folgende allgemeine Strukturformel aufweisen:

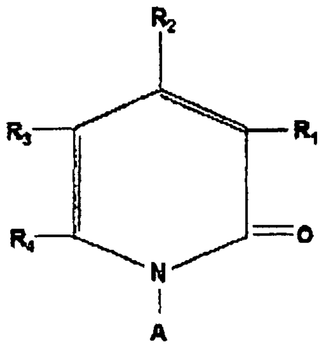

,

wobei $R_1$ eine Alkylgruppe ist, A ein Phenyl, ein Thienyl oder eine andere Arylgruppe ist, alternativ $R_3$ die Stelle der Substitution der Alkylgruppe ist, wobei $R_1$ als ein Wasserstoff verbleibt, und $R_2$ und $R_4$ unter allen Umständen Wasserstoffe sind.

6. Verwendung nach Anspruch 5, wobei die eine oder die mehreren 2-(1H)-Pyridon-Verbindungen aus der Gruppe ausgewählt sind, die besteht aus:

5-Methyl-1-phenyl-2-(1H)-pyridon
5-Methyl-1-(3-nitrophenyl-2)-(1H)-pyridon
5-Methyl-1-(4'-methoxyphenyl)-2-(1H)-pyridon
5-Methyl-1-p-tolyl-2-(1H)-pyridon
5-Methyl-1-(3'-trifluormethylphenyl)-2-(1H)-pyridon
1-(4'-Chlorphenyl)-5-methyl-2-(1H)-pyridon
5-Methyl-1-(2'-naphthyl)-2-(1H)-pyridon
5-Methyl-1-(1'-naphthyl)-2-(1H)-pyridon
3-Methyl-1-phenyl-2-(1H)-pyridon
3-Ethyl-1-phenyl-2-(1 H)-pyridon
6-Methyl-1-phenyl-2-(1H)-pyridon
3,6-Dimethyl-1-phenyl-2-(1 H)-pyridon
5-Methyl-1-(2'-thienyl)-2-(1H)-pyridon
1-(2'-Furyl)-5-methyl-2-(1H)-pyridon
5-Methyl-1-(5'-quinolyl)-2-(1H)-pyridon
5-Methyl-1-(4'-pyridyl)-2-(1H)-pyridon
5-Methyl-1-(3'-pyridyl)-2-(1H)-pyridon
5-Methyl-1-(2'-pyridyl)-2-(1H)-pyridon

5-Methyl-1-(2'-quinolyl)-2-(1H)-pyridon
5-Methyl-1-(4'-quinolyl)-2-(1H)-pyridon
5-Methyl-1-(2'-thiazolyl-2-(1H)-pyridon
1-(2'-Imidazolyl)-5-methyl-2-(1H)-pyridon
5-Ethyl-1-phenyl-2-(1H)-pyridon
1-Phenyl-2-(1H)-pyridon
1-(4'-Nitrophenyl)-2-(1H)-pyridon
1,3-Diphenyl-2-(1H)-pyridon
1-Phenyl-3-(4'-chlorphenyl)-2-(1H)-pyridon
1,3-Diphenyl-5-methyl-2-(1H)-pyridon
3-(4'-Chlorophenyl)-5-methyl-1-phenyl-2-(1H)-pyridon und
5-Methyl-3-phenyl-1-(2'-thienyl)-2-(1H)-pyridon

**7.** Verwendung nach einem der vorherigen Ansprüche, wobei die pharmazeutische Zusammensetzung die folgende Zusammensetzung in Gewichtsprozent aufweist:

| | |
|---|---|
| Pirfenidon (USAN) -Pulver | 5,0 |
| Propylenglycol 400 USP | 23,5 |
| Steriles destilliertes Wasser | 30,5 |
| Stearylalkohol USP | 20,5 |
| Weißes Petrolatum USP | 20,5 |
| | Gesamt: 100,0 |

**8.** Verwendung nach einem der Ansprüche 1 bis 6, wobei die pharmazeutische Zusammensetzung die folgende Zusammensetzung in Gewichtsprozent aufweist:

| | |
|---|---|
| Pirfenidon (USAN) -Pulver | 10,0 |
| Propylenglycol 400 USP | 14,3 |
| Steriles destilliertes Wasser | 41,7 |
| Stearylalkohol USP | 17,0 |
| Weißes Petrolatum USP | 17,0 |
| | Gesamt: 100,0 |

**9.** Verwendung nach einem der Ansprüche 1 bis 6, wobei die pharmazeutische Substanz die folgende Zusammensetzung in Gewichtsprozent aufweist:

| | |
|---|---|
| Pirfenidon (USAN) -Pulver | 50 g |
| Stearylsäure USP | 30 g |
| Emplilan SE 40* | 30 g |
| Isopropylmyristat | 30 g |
| Mineralöl | 115 g |
| Stearylalkohol USP | 5 g |
| Propylenglycol USP 400 | 50 g |
| Steriles destilliertes Wasser | 690 ml (690 g) |
| | Gesamt: 1000 g |

**10.** Verwendung nach einem der vorherigen Ansprüche, wobei die Zusammensetzung für die Behandlung von Infektionen ist, die durch Bakterien, Pilze und/oder Viren auf der Oberfläche oder innerhalb der Schichten der Dermis der Haut, der Ohren, der Fingernägel, der Zehennägel oder der Hufe der Säugetierspezies verursacht werden.

## Revendications

**1.** Utilisation d'un ou de plusieurs composé(s) 2-(1H)-pyridones dans la préparation d'une composition pharmaceutique topique destinée à une utilisation comme antiseptique.

2. Utilisation selon la revendication 1, dans laquelle le ou les composé(s) 2-(1H)-pyridones est (sont) présents dans une pommade, une crème ou une mousse.

3. Utilisation selon la revendication 2, dans laquelle ledit ou lesdits composé(s) 2-(1H)-pyridones est (sont) présents dans une dispersion aqueuse d'une ou de plusieurs substances.

4. Utilisation selon l'une quelconque des revendications précédentes, dans laquelle ledit ou lesdits composés 2-(1H)-pyridones sont présents en une concentration comprise dans la plage allant d'environ 2 % en poids à environ 10 % en poids de la composition.

5. Utilisation selon l'une quelconque des revendications précédentes, dans laquelle ledit ou lesdits composés 2-(1H)-pyridones ont la formule développée générale suivante :

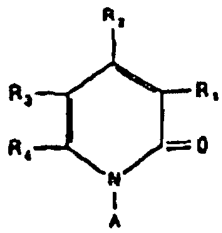

dans laquelle $R^1$ représente un groupe alkyle ; A représente un groupe phényle, un groupe thiényle ou un autre groupe aryle ; ou bien alternativement, $R_3$ représente le site de substitution dudit groupe alkyle, $R_1$ restant un atome d'hydrogène ; et $R_2$ et $R_4$ représentant, dans tous les cas, un atome d'hydrogène.

6. Utilisation selon la revendication 5, dans laquelle ledit ou lesdits composés 2-(1H)-pyridones sont choisis dans le groupe constitué des composés suivants :

5-méthyl-1-phényl-2-(1H)-pyridone,
5-méthyl-1-(3-nitrophényl)-2-(1H)-pyridone,
5-méthyl-1-(4'-méthoxyphényl)-2-(1H)-pyridone,
5-méthyl-1-p-tolyl-2-(1H)-pyridone,
5-méthyl-1-(3'-trifluorométhylphényl)-2-(1H)-pyridone,
1-(4'-chlorophényl)-5-méthyl-2-(1H)-pyridone,
5-méthyl-1-(2'-naphtyl)-2-(1H)-pyridone,
5-méthyl-1-(1'-naphtyl)-2-(1H)-pyridone,
3-méthyl-1-phényl-2-(1H)-pyridone,
3-éthyl-1-phényl-2-(1H)— pyridone,
6-méthyl-1-phényl-2-(1H)-pyridone,
3,6-diméthyl-1-phényl-2-(1H)-pyridone,
5-méthyl-1-(2'-thiényl)-2-(1H)-pyridone,
1-(2'-furyl)-5-méthyl-2-(1H)-pyridone,
5-méthyl-1-(5'-quinolyl)-2-(1H)-pyridone,
5-méthyl-1-(4'-pyridyl)-2-(1H)-pyridone,
5-méthyl-1-(3'-pyridyl)-2-(1H)-pyridone,
5-méthyl-1-(2'-pyridyl)-2-(1H)-pyridone,
5-méthyl-1-(2'-quinolyl)-2-(1H)-pyridone,
5-méthyl-1-(4'-quinolyl)-2-(1H)-pyridone,
5-méthyl-1-(2'-thiazolyl)-2-(1H)-pyridone,
1-(2'-imidazolyl)-5-méthyl-2-(1H)-pyridone,
5-éthyl-1-phényl-2-(1H)— pyridone,
1-phényl-2-(1H)— pyridone,

1-(4'-nitrophényl)-2-( 1H)—pyridone,
1,3-diphényl-2-(1H)—pyridone,
1-phényl-3-(4'-chlorophényl)-2-(1H)—pyridone,
1 ,3-diphényl-5-méthyl-2-( 1H)—pyridone,
3-(4'-chlorophényl)-5-méthyl-1-phényl-2-(1H)—pyridone, et
5-méthyl-3-phényl-1-(2'-thiényl)-2-(1H)-pyridone.

**7.** Utilisation selon l'une quelconque des revendications précédentes, dans laquelle ladite composition pharmaceutique a la composition suivante, en pourcentage en poids :

| | |
|---|---|
| Poudre de pirfénidone (USAN) | 5,0 |
| Propylène glycol 400 USP | 23,5 |
| Eau distillée stérile | 30,5 |
| Alcool stéarylique USP | 20,5 |
| Vaseline blanche USP | 20,5 |
| | TOTAL: 100.0 |

**8.** Utilisation selon l'une quelconque des revendications 1 à 6, dans laquelle ladite composition pharmaceutique a la composition suivante, en pourcentage en poids :

| | |
|---|---|
| Poudre de pirfénidone (USAN) | 10,0 |
| Propylène glycol 400 USP | 14,3 |
| Eau distillée stérile | 41,7 |
| Alcool stéarylique USP | 17,0 |
| Vaseline blanche USP | 17,0 |
| | TOTAL: 100.0 |

**9.** Utilisation selon l'une quelconque des revendications 1 à 6, dans laquelle ladite composition pharmaceutique a la composition suivante, en pourcentage en poids :

| | |
|---|---|
| Poudre de pirfénidone (USAN) | 50 g |
| Acide stéarique USP | 30 g |
| Empilan SE 40* | 30 g |
| Myristate d'isopropyle | 30 g |
| Huile minérale | 115 g |
| Alcool stéarylique USP | 5 g |
| Propylène glycol 400 USP | 50 g |
| Eau distillée stérile | 690 ml (690 g) |
| | TOTAL: 1000 g |

**10.** Utilisation selon l'une quelconque des revendications précédentes, dans laquelle la composition est destinée au traitement d'infections provoquées par des bactéries, des champignons et/ou des virus sur la surface de, ou dans les couches du derme de la peau, des oreilles, des ongles de la main, des ongles des pieds ou des sabots d'espèces mammifères.